(19) Europäisches Patentamt
European Patent Office
Office européen des brevets

(11) EP 1 293 193 B1

(12) # FASCICULE DE BREVET EUROPEEN

(45) Date de publication et mention de la délivrance du brevet:
**02.11.2005 Bulletin 2005/44**

(51) Int Cl.[7]: **A61K 7/48**

(21) Numéro de dépôt: **02292089.6**

(22) Date de dépôt: **22.08.2002**

(54) **Utilisation de l'acide pantéthéine sulfonique et/ou de ses sels en tant qu'agent anti-radicalaire**

Verwendung von Pantethein-Sulfonsäure und/oder deren Salzen als anti-radikal Agent

Use of pantetheine sulfonic acid and/or salts thereof as anti-radical agent

(84) Etats contractants désignés:
**AT BE BG CH CY CZ DE DK EE ES FI FR GB GR IE IT LI LU MC NL PT SE SK TR**

(30) Priorité: **17.09.2001 FR 0111993**

(43) Date de publication de la demande:
**19.03.2003 Bulletin 2003/12**

(73) Titulaire: **L'OREAL**
**75008 Paris (FR)**

(72) Inventeurs:
- **Potin, Anthony**
  **75006 Paris (FR)**
- **Touzan, Philippe 52**
  **75012 Paris (FR)**
- **Pelletier, Pascale**
  **92160 Antony (FR)**

(74) Mandataire: **Renard, Emmanuelle**
**L'OREAL - D.I.P.I.**
**25-29 Quai Aulagnier**
**92600 Asnières (FR)**

(56) Documents cités:
**WO-A-00/57840**
**WO-A-01/91715**
**DE-A- 3 244 806**
**FR-A- 2 532 175**
**US-A- 4 749 719**
**US-A- 5 601 806**
**US-A- 5 723 109**

- **DATABASE CAPLUS [en ligne] CHEMICAL ABSTRACTS SERVICE, COLUMBUS, OHIO, US; retrieved from STN Database accession no. 1997:4511 XP002203354 & JP 08 259420 A (LION CORP) 8 octobre 1996 (1996-10-08)**
- **DATABASE CAPLUS [en ligne] CHEMICAL ABSTRACTS SERVICE, COLUMBUS, OHIO, US; retrieved from STN Database accession no. 1983:149594 XP002203355 & JP 03 054081 A (SOGO YOKUKO CO) 8 mars 1991 (1991-03-08)**

EP 1 293 193 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention se rapporte à l'utilisation de l'acide pantéthéine sulfonique et/ou de l'un de ses sels en tant qu'agent anti-radicalaire, notamment pour prévenir ou traiter les signes du vieillissement cutané actinique.

Au cours du temps, il apparaît différents signes sur la peau, très caractéristiques du vieillissement, se traduisant notamment par une modification de la structure et des fonctions cutanées.

Ce vieillissement, de nature physiologique, peut-être accéléré par des facteurs environnementaux tels qu'une exposition répétée de la peau à la lumière solaire, et notamment aux rayonnements ultraviolets A, à la pollution, en particulier ou aux particules de diesel ou à la fumée de cigarette. L'action de l'environnement sur les constituants de la peau (fibres, cellules, enzymes) et sur le sébum sécrété par la peau entraîne en particulier la formation de radicaux libres oxygénés. Or, ces radicaux provoquent des dégâts oxydatifs importants, notamment dans les membranes cellulaires (perméabilité des membranes), les noyaux des cellules (destruction de l'ADN), et les tissus, en particulier le tissu conjonctif (dégradation des fibres d'élastine et de collagène). Ces dégâts conduisent notamment à une perte de fermeté et d'élasticité de la peau.

Il a en outre été suggéré que les radicaux libres pouvaient intervenir dans le processus de fabrication de la mélanine conduisant à la pigmentation de la peau.

Le mécanisme de formation de la mélanine est particulièrement complexe et fait intervenir schématiquement les principales étapes suivantes :

Tyrosine → Dopa → Dopaquinone → Dopachrome → Mélanine

La tyrosinase (monophénol dihydroxyl phénylalanine : oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce à son activité hydroxylase. Or, certains auteurs pensent que l'étape d'hydroxylation de la tyrosine en Dopa pourrait être initiée par des radicaux OH° (C. Montastier et al., Méthodes d'objectivation des effets des agents dépigmentants chez l'homme, J. Med. Esth. et Chir. Derm., Vol. XXII, 86, Juin 1995, pp. 93-103).

Les radicaux libres formés sous l'effet de facteurs environnementaux pourraient donc entraîner une augmentation de la formation de la mélanine, et provoquer ou accentuer ainsi certaines hyperpigmentations indésirables telles que les mélasmas idiopathiques, survenant lors de la grossesse ("masque de grossesse" ou chloasma) ou d'une contraception oestro-progestative ou encore les hyperpigmentations localisées, telles que les taches pigmentaires séniles dites lentigo actiniques.

Il est donc nécessaire de protéger la peau contre ces radicaux libres.

Cette fonction protectrice est normalement assurée par des enzymes présentes dans le tissu cutané. Toutefois, dans certaines circonstances, le rôle de ces enzymes n'est pas suffisant pour bloquer totalement l'action destructrice des radicaux libres.

Il a donc été proposé d'utiliser différents produits naturels et synthétiques comme actifs cosmétiques permettant de lutter contre la formation de radicaux libres. Parmi ceux-ci, on peut citer :

- les composés piégeurs de radicaux libres tels que la vitamine C, la vitamine E et leurs dérivés, ainsi que les caroténoïdes ;
- les composés stimulant les enzymes bloquant les radicaux libres, tels que les composés riches en phosphore et les oligo-éléments ;
- les composés maintenant l'intégrité du film hydrolipidique ayant une fonction d'auto-protection contre les rayonnements générateurs de radicaux libres. Il s'agit en particulier du gamma-oryzanol extrait notamment de l'huile de son de riz.

La Demanderesse a maintenant découvert, de façon surprenante, que l'acide pantéthéine sulfonique et/ou l'un de ses sels avait des propriétés anti-radicalaires permettant d'envisager son utilisation dans des compositions, notamment cosmétiques, destinées à protéger la peau contre les effets des UV et de la pollution en particulier contre le photo-vieillissement, mais également dans des compositions blanchissantes ou dépigmentantes.

Par "pollution", on entend aussi bien la pollution "extérieure", due par exemple aux particules de diesel, à l'ozone ou aux métaux lourds, que la pollution "intérieure" qui peut notamment être due aux émissions de solvants de peinture, de colles de moquette, d'isolants ou de papiers peints (tels que toluène, styrène, xylène ou benzaldéhyde), ou bien à la fumée de cigarette. Tous ces polluants sont en effet susceptibles de générer, directement ou indirectement, des radicaux libres.

L'acide pantéthéine sulfonique et ses sels ont notamment été décrits comme agents favorisant la repousse

des cheveux (JP-09 110 645), comme agents anti-pigmentants ou destinés à blanchir la peau (JP-09 059 142 et WO 00/57840), notamment par inhibition de l'activité de la tyrosinase (FR-2 532 175, WO 01/91715 et US-4,749,719), et à augmenter le renouvellement cellulaire, en particulier dans des compositions anti-âge contenant par ailleurs un hydroxy-acide (JP-08 259 420). Il a également été suggéré de les utiliser pour inhiber l'irritation de la peau (JP-03 054 081). Enfin, les sels d'acide pantéthéine sulfonique ont été décrits comme adjuvants dans des compositions contenant des analogues de taurine en tant qu'agents anti-radicalaires (US-5,601,806).

Toutefois, à la connaissance de la Demanderesse, il n'a encore jamais été suggéré que l'acide pantéthéine sulfonique et/ou ses sels pouvaient posséder des propriétés anti-radicalaires.

La présente invention a donc pour premier objet l'utilisation cosmétique de l'acide pantéthéine sulfonique et/ou de l'un de ses sels dans une composition renfermant un milieu physiologiquement acceptable, en tant qu'agent anti-radicalaire.

La présente invention a pour second objet l'utilisation cosmétique de l'acide pantéthéine sulfonique et/ou de l'un de ses sels dans une composition renfermant un milieu physiologiquement acceptable, pour protéger la peau contre le photo-vieillissement.

Bien que tout sel alcalin ou alcalino-terreux d'acide pantéthéine sulfonique convienne à une utilisation dans la présente invention, on préfère utiliser le sel de calcium de l'acide pantéthéine sulfonique.

La composition est de préférence adaptée à une application topique sur la peau et comprend donc généralement un milieu physiologiquement acceptable, c'est-à-dire compatible avec la peau et/ou ses phanères.

La quantité d'acide pantéthéine sulfonique ou de son sel dans la composition selon l'invention est fonction de l'effet recherché et peut par exemple aller de 0,001 à 10% en poids, et de préférence de 0,01 à 5% en poids, par rapport au poids total de la composition.

La composition renfermant l'acide pantéthéine sulfonique ou son sel peut notamment être destinée à prévenir ou traiter les signes du vieillissement cutané, en particulier du photo-vieillissement et plus particulier la perte de fermeté et/ou d'élasticité de la peau.

L'invention a donc aussi pour objet l'utilisation cosmétique de l'acide pantéthéine sulfonique et/ou de l'un de ses sels dans une composition renfermant un milieu physiologiquement acceptable, pour prévenir ou traiter la perte de fermeté et/ou d'élasticité de la peau.

La composition est, de préférence adaptée à une application topique sur la peau. Elle peut se présenter sous toutes les formes galéniques normalement utilisées pour ce type d'application, notamment sous forme d'une solution aqueuse ou huileuse, d'une émulsion huile-dans-eau ou eau-dans-huile ou multiple, d'une émulsion siliconée, d'une microémulsion ou nanoémulsion, d'un gel aqueux ou huileux ou d'un produit anhydre liquide, pâteux ou solide.

Cette composition peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage de la peau.

De façon connue, la composition peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse ou dans la phase aqueuse. Ces adjuvants, ainsi que leurs concentrations, doivent être tels qu'ils ne nuisent pas aux propriétés avantageuses de l'acide pantéthéine sulfonique ou de ses sels.

Ainsi, la composition renferme avantageusement au moins un filtre UVA et/ou UVB choisi parmi les filtres organiques et les filtres inorganiques.

Des exemples de filtres UVA organiques convenant à une utilisation dans cette invention comprennent notamment :

(1) les dérivés de benzophénone, les benzophénones 3 et 5 étant préférées ;

(2) les dérivés de triazine, et en particulier la 2,4-bis {[4-(2-éthyl-hexyloxy)-2-hydroxy]-phényl}-6-(4-méthoxy-phényl)-1,3,5-triazine disponible auprès de la société CIBA GEIGY sous la dénomination commerciale TINOSORB S et le 2,2'-méthylènebis-[6-(2H benzotriazol-2-yl)4-(1,1,3,3-tétraméthylbutyl)phénol] disponible auprès de la société CIBA GEIGY sous la dénomination commerciale TINOSORB M ;

(3) l'acide téréphtalylidène dicamphre sulfonique ou acide benzène 1,4 [di(3-méthylidènecampho 10-sulfonique)] ou l'un de ses sels de métal alcalin ou alcalino-terreux, d'ammonium ou avec une base organique, qui répond à la formule (I) suivante

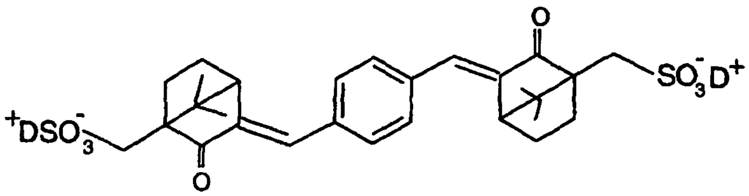

(I)

dans laquelle D désigne un atome d'hydrogène, un métal alcalin ou encore un radical $NH(R_{25})_3^+$ dans lequel les radicaux $R_{25}$, qui peuvent être identiques ou différents, désignent un atome d'hydrogène, un radical alkyle ou hydroxyalkyle en $C_1$-$C_4$ ou encore un groupement $M^{n+}/n$, $M^{n+}$ désignant un cation métallique polyvalent dans lequel n est égal à 2 ou 3 ou 4, $M^{n+}$ désignant de préférence un cation métallique choisi parmi $Ca^{2+}$, $Zn^{2+}$, $Mg^{2+}$, $Ba^{2+}$, $Al^{3+}$ et $Zr^{4+}$ ; et
(4) leurs mélanges.

L'acide téréphtalylidène dicamphre sulfonique est préféré pour une utilisation dans cette invention.
Comme filtres UVB organiques, on peut notamment citer :

(1) les dérivés de l'acide salicylique, en particulier le salicylate d'homomenthyle et le salicylate d'octyle ;
(2) les dérivés de l'acide cinnamique, en particulier le p-méthoxycinnamate de 2-éthylhexyle, disponible auprès de la société GIVAUDAN sous la dénomination commerciale Parsol MCX ;
(3) les dérivés de β,β'-diphénylacrylate liquides, en particulier l'α-cyano-α,β' diphénylacrylate de 2-éthylhexyle, ou octocrylène, disponible auprès de la société BASF sous la dénomination commerciale UVINUL N539;
(4) les dérivés de l'acide p-aminobenzeïque ;
(5) le 4-méthyl benzylidène camphre disponible auprès de la société MERCK, sous la dénomination commerciale EUSOLEX 6300 ;
(6) l'acide 2-phénylbenzimidazole 5-sulfonique vendu sous la dénomination commerciale EUSOLEX 232 par la société MERCK
(7) les dérivés de 1,3,5-triazine, en particulier :

- la 2,4,6-tris[p-(2'-éthylhexyl-1'-oxycarbonyl)anilino]-1,3,5-triazine, disponible auprès de la société BASF sous la dénomination commerciale UVINUL T150, et
- le composé répondant à la formule (II) suivante :

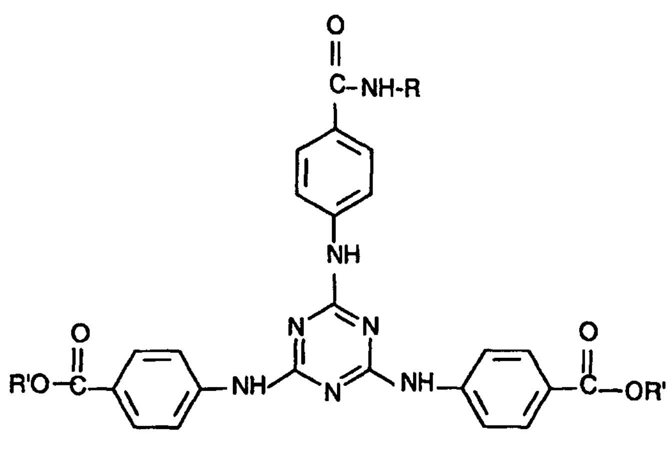

(II)

dans laquelle R' désigne un radical éthyl-2 hexyle et R désigne un radical tert- butyle, disponible auprès de la société SIGMA 3V sous la dénomination commerciale UVASORB HEB ; et (8) leurs mélanges.
On préfère utiliser les filtres (2) et (6)
Un exemple de filtre UVA et UVB (filtre à large bande) est constitué par la silicone benzotriazole répondant à la formule générale (III) suivante :

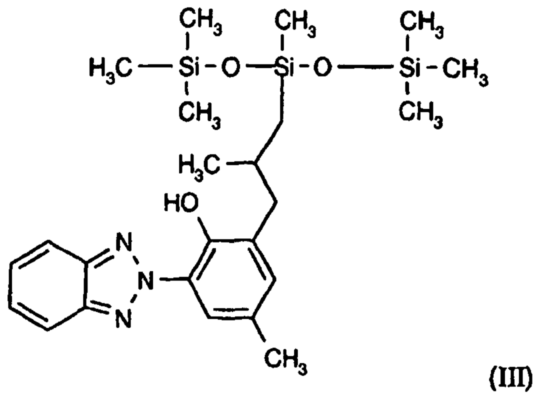

(III)

Cette silicone benzotriazole, ainsi que son mode de préparation, sont décrits notamment dans la demande FR-A-2 642 968.

Les filtres inorganiques utilisables dans la composition selon l'invention sont en particulier les nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

En variante, la composition contenant l'acide pantéthéine sulfonique ou son sel peut comprendre d'autres agents anti-pollution, qu'il s'agissent d'agents susceptibles de piéger les radicaux libres, l'ozone, les composés aromatiques ou les métaux lourds (cobalt, mercure, cadmium et/ou nickel).

Comme agents anti-pollution utilisables dans la composition selon l'invention, on peut citer en particulier la vitamine C et ses dérivés dont le glucoside d'ascorbyle ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les extraits de marc et de pépins de raisin ; les anthocyanes ; les extraits de romarin ; les acides phénols, en particulier l'acide chlorogénique ; les stilbènes, en particulier le resvératrol ; les acides aminés soufrés tels que la cystéine ; les dérivés d'acides aminés soufrés, en particulier la S-carboxyméthylcystéine ; l'ergothionéine ; la N-acétylcystéine ; des chélatants comme l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; des caroténoïdes tels que la crocétine ; des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F®, la fraction hydrosoluble de maïs commercialisée par la société SOLABIA sous la dénomination commerciale Phytovityl®, le mélange d'extrait de fumeterre et d'extrait de citron commercialisé sous la dénomination Unicotrozon C-49® par la société Induchem, ou le mélange d'extraits de ginseng, de pomme, de pêche, de blé et d'orge vendu par la société PROVITAL sous la dénomination commerciale Pronalen Bioprotect® ; les extraits de Jacinthe d'eau ou eichornia crassipes ; l'acide phytique ; les dérivés de chitosan ; la vitamine E et ses dérivés tels que l'acétate de tocophéryle ; les bioflavonoïdes ; le co-enzyme Q10 ou ubiquinone ; certaines enzymes comme la catalase, le superoxyde dismutase, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; les lignanes ; et la mélatonine.

Lorsque la composition est une émulsion, la proportion de la phase grasse peut aller de 5 à 80 % en poids, et de préférence de 5 à 50 % en poids par rapport au poids total de la composition. Les matières grasses, les émulsionnants et les coémulsionnants utilisés dans la composition sous forme d'émulsion sont choisis parmi ceux classiquement utilisés dans le domaine considéré. L'émulsionnant et le coémulsionnant sont de préférence présents, dans la composition, en une proportion allant de 0,3 à 30 % en poids, et de préférence de 0,5 à 20 % en poids par rapport au poids total de la composition.

Comme matières grasses utilisables dans l'invention, on peut utiliser les huiles et notamment les huiles minérales (huile de vaseline), les huiles d'origine végétale (huile d'avocat, huile de soja), les huiles d'origine animale (lanoline), les huiles de synthèse (octyldodécanol), les huiles siliconées (cyclométhicone et diméthicone) et les huiles fluorées (perfluoropolyéthers). On peut aussi utiliser comme matières grasses des alcools gras tels que l'alcool cétylique, des acides gras tels que l'acide stéarique, des cires et des gommes et en particulier les gommes de silicone.

Comme émulsionnants et coémulsionnants utilisables dans l'invention, on peut citer par exemple les esters d'acide gras et de polyéthylène glycol tels que le stéarate de PEG-100, le stéarate de PEG-50 et le stéarate de PEG-40 ; les esters d'acide gras et de polyol tels que le stéarate de glycéryle, le tristéarate de sorbitane et les stéarates de sorbitane oxyéthylénés disponibles sous les dénominations commerciales Tween® 20 ou Tween® 60, par exemple ; les esters de sucre tels que le méthyl glucose sesquistéarate et/ou son dérivé oxyéthyléné ; et leurs mélanges.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

L'invention sera maintenant illustrée par les exemples non limitatifs suivants. Dans ces exemples, les quantités sont indiquées en pourcentage pondéral.

**EXEMPLES**

**Exemple 1 : effet antiradicalaire du pantéthéine sulfonate de calcium**

L'effet antiradicalaire du pantéthéine sulfonate de calcium a été mis en évidence par le test dit « test éthylène » basé sur la mesure en chromatographie en phase gazeuse de l'éthylène formé à partir de l'oxydation de la méthionine par le radical hydroxyle.

Le principe de ce test est décrit dans la publication "Ethylene Formation from Methionine as a Method to Evaluate Oxygen Free-Radical Scavenging and Metal Inactivation by Cosmetics", J.B. Galey, F. Millecamps et Q.L. NGuyen, International Journal of Cosmetic Science, 13, 65-78 (1991).

Le Calcium D-Pantéthéine-S-Sulfonate utilisé a été obtenu auprès de la société SOGO PHARMACEUTICAL CO. sous forme de solution aqueuse à 70% de matière active.

Il a été étudié en effet dose entre 0,1 % et 1,5 % (p/v en matière première dans le milieu réactionnel, soit 0,07 % à 1,05 % p/v de matière active).

Les résultats obtenus sont récapitulés dans le tableau suivant :

| **Concentration en Calcium D- Pantéthéine-S-Sulfonate (%)** | **Inhibition de la production d'éthylène par rapport au témoin** (%) |
|---|---|
| 0,1 | 31,1 |
| 0,5 | 53,6 |
| 1 | 56,6 |
| 1,5 | 61,0 |

Les résultats obtenus montrent que le Calcium D-Pantéthéine-S-Sulfonate possède un pouvoir inhibiteur dose dépendant vis-à-vis de la production d'éthylène. Celle-ci étant proportionnelle à la production de radicaux OH°, ces résultats montrent clairement l'effet antiradicalaire de ce composé.

**Exemple 2 : fluide de soin SPF15** (hors invention)

| | | |
|---|---|---|
| **A -** | Eau | 53 % |
| | Glycérine | 3 % |
| | Conservateurs | 0,5 % |
| | EDTA trisodique | 0,05% |
| **B -** | Cyclohexasiloxane | 7 % |
| | Stéarate de glycéryle, stéarate de PEG-100, polysorbate 60, alcool cétylique et acide stéarique | 3,8 % |
| | Ethylhexyl méthoxycinnamate | 7,5 % |

(suite)

| | | |
|---|---|---|
| | Conservateurs | 0,15% |
| | Parfum | 0,1 % |
| C - | Eau | 10 % |
| | Ammonium polyacryloyldiméthyl taurate | 1 % |
| D - | Eau | qsp 100 % |
| | Terephtalidene dicamphor Sulfonic Acid | 0,7 % |
| | Phenylbenzimidazole sulfonic acid | 2 % |
| | Triethanolamine | qsp pH 6,5 |
| E - | Acrylate copolymer | 0,3 % |
| F - | Calcium D-Pantéthéine-S-Sulfonate à 70% en solution aqueuse | 1 % |

La composition ci-dessus peut être obtenue de la façon suivante.

La phase A est chauffée sous agitation à 80°C jusqu'à parfaite solubilisation. La phase B est chauffée sous agitation à 80°C jusqu'à obtention d'une phase limpide puis ajoutée à la phase A sous agitation. Le mélange est ensuite refroidi à 60°C. L'ammonium polyacryloyldimethyl taurate est mis à gonfler à 60°C dans l'eau pendant 10 minutes et la phase C est ajoutée au mélange des phases A + B. La phase D est solubilisée sous agitation à 50°C avant d'être ajoutée au mélange des phases A + B + C. Le tout est ensuite ramené à 30°C. Les phases E et F sont successivement introduites à 30°C. La température du mélange est ensuite ramenée à 20°C.

Ce fluide peut être utilisé en applications quotidiennes pour prévenir les effets nuisibles des UV et de la pollution sur la peau.

**Exemple 3 : crème blanchissante** (hors invention)

La composition ci-dessous est préparée de façon classique pour l'homme du métier.

| | |
|---|---|
| Octyldodécanol | 1 % |
| Polysorbate 60 | 0,7 % |
| Acide stéarique | 0,5 % |
| Stéarate de glycéryle et stéarate de PEG-100 | 1,6 % |
| EDTA disodique | 0,2 % |
| Neutralisants | 0,2 % |
| Gélifiants | 2,0 % |
| Glycérine | 3 % |
| Conservateurs | 0,5 % |
| Acide n-octanoyl-5-salicylique | 0,1 % |
| Alcool cétylique | 1 % |
| Cyclohexasiloxane | 1 % |
| Glucoside d'ascorbyle | 0,05% |
| Calcium pantéthéine sulfonate à 70% en solution aqueuse | 0,1 % |
| Eau | qsp 100 % |

Cette crème peut être utilisée en applications bi-quotidiennes pour blanchir la peau et l'aider à retrouver sa transparence.

## Revendications

1. Utilisation cosmétique de l'acide pantéthéine sulfonique et/ou de l'un de ses sels dans une composition renfermant un milieu physiologiquement acceptable, en tant qu'agent anti-radicalaire.

2. Utilisation cosmétique de l'acide pantéthéine sulfonique et/ou de l'un de ses sels dans une composition renfermant

un milieu physiologiquement acceptable, pour protéger la peau contre le photo-vieillissement.

3. Utilisation cosmétique de l'acide pantéthéine sulfonique et/ou de l'un de ses sels dans une composition renfermant un milieu physiologiquement acceptable, pour prévenir ou traiter la perte de fermeté et/ou d'élasticité de la peau.

4. Utilisation selon l'une quelconque des revendications 1 à 3, **caractérisée en ce que** le sel d'acide pantéthéine sulfonique est un sel de métal alcalin ou alcalino-terreux d'acide pantéthéine sulfonique.

5. Utilisation selon la revendication 4, **caractérisée en ce que** le sel d'acide pantéthéine sulfonique est le sel de calcium de l'acide pantéthéine sulfonique.

6. Utilisation selon l'une quelconque des revendication 1 à 5, **caractérisée en ce que** la composition est adaptée à une application topique sur la peau.

## Patentansprüche

1. Kosmetische Verwendung der Pantetheinsulfonsäure und/oder eines ihrer Salze als Radikalfänger in einer Zusammensetzung, die ein physiologisch akzeptables Medium enthält.

2. Kosmetische Verwendung der Pantetheinsulfonsäure und/oder eines ihrer Salze in einer Zusammensetzung, die ein physiologisch akzeptables Medium enthält, zum Schutz der Haut gegen lichtinduzierte Alterung.

3. Kosmetische Verwendung der Pantetheinsulfonsäure und/oder eines ihrer Salze in einer Zusammensetzung, die ein physiologisch akzeptables Medium enthält, um dem Verlust der Festigkeit und/oder der Elastizität der Haut vorzubeugen und/ oder ihn zu behandeln.

4. Verwendung nach einem der Ansprüche 1 bis 3, **dadurch gekennzeichnet, dass** das Salz der Pantetheinsulfonsäure ein Alkalimetallsalz oder Erdalkalimetallsalz der Pantetheinsulfonsäure ist.

5. Verwendung nach Anspruch 4, **dadurch gekennzeichnet, dass** das Salz der Pantetheinsulfonsäure das Calciumsalz von Pantetheinsulfonsäure ist.

6. Verwendung nach einem der Ansprüche 1 bis 5, **dadurch ge kennzeichnet, dass** die Zusammensetzung für eine topische Anwendung auf die Haut geeignet ist.

## Claims

1. Cosmetic use of pantethinesulphonic acid and/or one of its salts in a composition containing a physiologically acceptable medium, as a free-radical scavenger.

2. Cosmetic use of pantethinesulphonic acid and/or one of its salts in a composition containing a physiologically acceptable medium, to protect the skin against light-induced ageing.

3. Cosmetic use of pantethinesulphonic acid and/or one of its salts in a composition containing a physiologically acceptable medium, to prevent or treat the loss of firmness and/or elasticity of the skin.

4. Use according to any one of Claims 1 to 3, **characterized in that** the salt of pantethinesulphonic acid is an alkali metal or alkaline-earth metal salt of pantethinesulphonic acid.

5. Use according to Claim 4, **characterized in that** the salt of pantethinesulphonic acid is the calcium salt of pantethinesulphonic acid.

6. Use according to any one of Claims 1 to 5, **characterized in that** the composition is suitable for topical application to the skin.